# EUROPEAN PATENT APPLICATION

(11) **EP 3 906 922 A1**
(43) Date of publication of application: **10.11.2021**
(21) Application number: 20173595.8
(22) Date of filing: 07.05.2020
(51) Int. Cl.: A61K 31/395, A61P 11/00

(54) **NEW COMPOSITIONS AND METHODS OF TREATING COVID-19 DISEASE**

(71) Applicant: 4Living Biotech, 59000 Lille (FR); UNIVERSITE DE BORDEAUX, 33000 Bordeaux (FR); CENTRE HOSPITALIER DE BORDEAUX, 33404 Talence (FR); INSTITUT NATIONAL DE LA SANTE ET DE LA RECHERCHE MEDICALE - INSERM, 75013 Paris (FR)
(72) Inventor: BERGER, Patrick, 33700 Mérignac (FR); DUPIN, Isabelle, 33400 TALENCE (FR); PREVEL, Renaud, 33800 BORDEAUX (FR); JAMES, Chloé, 33600 Pessac (FR); GIRODET, Pierre-Olivier, 33200 BORDEAUX (FR); BISMUTH, Keren, 75011 Paris (FR); RATTENBACH, Revital, 75004 Paris (FR)
(74) Representative: Lecca, Patricia S.

(57) **Abstract**

The present invention relates novel composition to for use in a method of treating COVID-19 comprising administering to a patient a therapeutically effective amount of at least one antagonist or inhibitor of chemokine receptor CXCR4.

## Description

### FIELD OF INVENTION

The invention relates to novel compositions and methods for the treatment of COVID-19 disease. More specifically, the invention relates to the use of at least one antagonist or inhibitor of chemokine receptor CXCR4 for the treatment of COVID-19 in a patient at moderate to severe stages of the disease.

### BACKGROUND OF THE INVENTION

Coronaviruses are known to cause severe respiratory and gastrointestinal diseases in animals. The infection of humans with coronavirus strains have been described for many years to be associated with respiratory tract infections *i.e.* common cold-like diseases. For example, SARS-CoV (Severe Acute Respiratory Syndrome-Corona Virus) is a highly aggressive human agent, causing acute respiratory distress syndrome (ARDS), with often fatal outcome. This virus appeared as an epidemic in 2003 after having crossed the species barriers from bats to civet cats and humans demonstrating the potential of coronaviruses to cause high morbidity and mortality in humans. The strains HCoV-NL63 and HCoV-HKU1 were discovered in 2004 and 2005, respectively. In 2012, anew human CoV MERS (Middle East Respiratory Syndrome virus, previously called "EMC") emerged from the Middle East with clinical outcomes such as renal failure and acute pneumonia, similar to those of SARS-CoV but with an even higher mortality rate of about 50%.

On 31 December 2019, the World Health Organization (WHO) China Country Office was informed of cases of pneumonia of unknown etiology detected in Wuhan City, Hubei Province of China. From 31 December 2019 through 3 January 2020, a total of 44 case-patients with pneumonia of unknown etiology were reported to WHO by the national authorities in China. During this reported period, the causal agent was not identified. By 1^{st} March 2020, 44 672 patients in China were reported with confirmed infection, 2.1% being below the age of 20. The most commonly reported symptoms included fever, dry cough, and shortness of breath, and most patients (80%) experienced mild illness. Approximately 14% experienced severe disease and 5% were critically ill. Early reports suggested that illness severity is associated with age (>60 years old) and co-morbid diseases. As on April 29^{th}, 2020 there were 3,152,556 confirmed cases worldwide, with 218,491 reported dead due to COVID-19.

Severe acute respiratory syndrome-coronavirus 2 (SARS-CoV-2) has been identified as the emerging virus responsible for the pandemic COVID-19 now declared as a "global threat for public health" by the WHO. In most cases, clinical features of COVID-19 remain benign, however COVID-19 condition can be much more severe and may require for oxygen support at hospital in 16 to 20% of patients. It can cause acute respiratory distress syndrome (ARDS) in 5 to 10% of patients with admission to intensive care unit (ICU) and invasive mechanical ventilation. Long ICU length of stay participates in healthcare resources overwhelming. ARDS is responsible for 99% of admission to ICU and COVID-19 related deaths. ARDS is characterized by a respiratory worsening triggered by gas exchange impairment secondary to alveolar-capillary barrier oedema. This lung injury results from a massive dysregulated inflammatory response to lung epithelial invasion by the virus.

Therefore, it is an object of the present disclosure to provide new compositions and methods for the treatment of COVID-19 which are expected to be especially effective in improving survival rate in moderate to severe cases. Surprisingly, it has been found that therapeutically effective amount of at least one antagonist or inhibitor of chemokine receptor CXCR4 provides critical support and health improvement in COVID-19 patients.

### SUMMARY OF THE INVENTION

The present application relates to a composition for use in a method of treating and to a method of treating COVID-19 comprising administering to a patient a therapeutically effective amount at least one antagonist or inhibitor of chemokine receptor CXCR4.

Said compositions and methods of treatment are particularly effective in improving survival in moderate to severe cases of COVID-19, as well as in cases of co-morbidity or multiple co-morbidities.

### BRIEF DESCRIPTION OF THE FIGURES

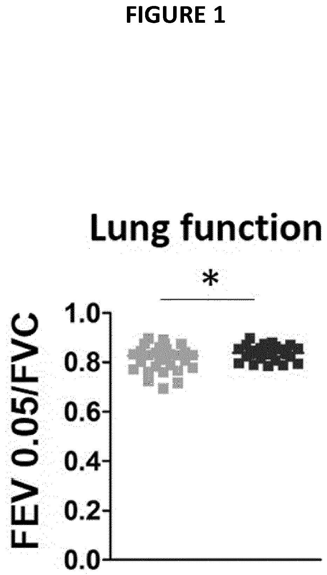

**Figure 1****:** is a graph showing murine lung function measured by the FEV0.05/FVC after daily subcutaneous injections of 1 mg/kg plerixafor during the 5 last weeks of the protocol. Mice tested presented lung injury induced by cigarette smoke exposure with poly-IC instillations, and either PBS injections (gray squares) or 1 mg/kg plerixafor injections (black squares). *: P<0.05.

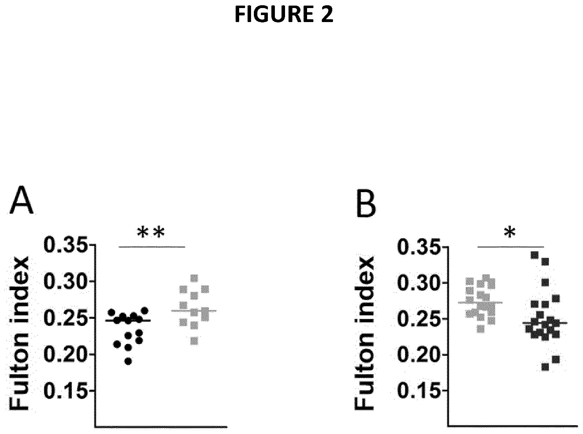

**Figure 2****:** are graphs showing cardiac remodeling, quantified by Fulton index, calculated as RV/(LV+S), with LV: left ventricle, RV: right ventricle, S: septum. (A) Fulton index. Control mice (black circles) and mice with lung injury induced by cigarette smoke exposure, with poly-IC instillations (gray squares). (B) Fulton index. Tested mice presented lung injury induced by cigarette smoke exposure with poly-IC instillations, and either PBS injection (gray squares) or 1 mg/kg plerixafor injection (black squares). *: P<0.05, **: P<0.01.

### DETAILED DESCRIPTION

The present invention thus provides compounds, compositions, pharmaceutical compositions and methods of use of certain compounds that are antagonists or inhibitors of chemokine receptor CXCR4, for treating coronavirus infections in general and more particularly COVID-19 infection.

More specifically the present invention provides a composition for use in a method of treating and a method of treating COVID-19 with or without multiple comorbidities comprising administering to said patient a therapeutically effective amount at least one antagonist or inhibitor of chemokine receptor CXCR4.

As used herein, the term "treatment" or "treat" refer to curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to cure, delay the onset of, reduce the severity of, or ameliorate one or more symptoms of a disorder or recurring disorder, or in order to prolong the survival of a subject beyond that expected in the absence of such treatment.

According to the present invention, antagonist or inhibitor of chemokine receptor CXCR4 may be chosen among indole-based compound, a N-substituted indole compound, a bicyclam compound, a cyclam mimetic compound, a para-xylyl-enediamine-based compound, a guanidine-based antagonist compound, a tetrahydroquinolines-based compound, or a 1,4-phenylenebis(methylene) compound.

Preferred antagonist or inhibitor of chemokine receptor CXCR4 is chosen among plerixafor (AMD3100), Burixafor (TG-0054), JM1657, AMD3329, AMD3465, AMD070, MSX-122, CTCE-9908, WZ811, or BKT-140.

Most preferred antagonist or inhibitor of chemokine receptor CXCR4 according to the present invention is plerixafor.

Plerixafor is well known drug and a CXCR4 antagonist, which has originally been developed as an anti-HIV drug. Also, the combination plerixafor and granulocyte-colony stimulating factor (G-CSF) was approved in 2008, by the US Food and Drug Administration to mobilize hematopoietic stem cells (HSCs) to the peripheral blood for collection and subsequent autologous transplantation in patients with non-Hodgkin's lymphoma and multiple myeloma. Plerixafor has been validated thru several clinical trials. Toxicology, pharmacokinetics as well as any potential adverse events are thus fully known, thereby allowing possible fast track of the registration for COVID-19 condition.

Applicants have showed in the Example below that the administration of a therapeutically effective amount of CXCR4 antagonist, such as for example plerixafor (1 mg/kg) during the 5 weeks improved lung function of mice having lung injury induced by cigarette smoke exposure with poly-IC instillations, which mimic viral infection, and either PBS injection (Figure 1).

This is mounting evidence strongly suggesting that plerixafor may significantly improve respiratory condition and lung disease evolution of COVID-19 patients at moderate or severe stages. The administration of a therapeutically effective amount of CXCR4 antagonist or inhibitor as described above is thus expected to decrease ICU length of stay, mortality in critically ill COVID-19 patients.

This is particularly surprising since plerixafor has been disqualified in February 24, 2020 article published by Hosseini FS et al. on the platform preprints.org (doi: 10.20944/preprints202002.0438.v1). In this article, the authors performed a virtual screen to identify FDA-approved small molecules that present an inhibitory potential of the SARS-CoV-2 protease, based on a simulation of molecular docking and predicted binding energy within the binding pocket of the virus' protease. The authors expressly rejected clinical exploration of plerixafor for patients with COVID-19 given plerixafor known side effect of bone marrow and strong immune system suppression which was expected to lead to a worsening of the condition of the patients.

Composition and methods according to the present invention are efficient for treating COVID-19 patients at moderate to advanced or severe stage of the disease.

Moderate COVID-19 cases are those with inflammation lower down in the lungs, so lung symptoms like cough are more marked. The lungs consist of large airways (bronchi), smaller airways (bronchioles) and the tiny air sacs on the end (alveoli) where oxygen is extracted from the air. They contain a fluid called surfactant which keeps the lungs stretchy and compliant and helps keep the air sacs open. Patients with moderate COVID-19 may have inflammation moving down into the bronchioles. They are more breathless and tend to have an increased heart rate, particularly if they are moving around.

While the majority of patients with COVID-19 have benign or mild or moderate illness, 16 to 20% of affected patients develop severe COVID-19 conditions causing acute respiratory distress syndrome (ARDS). Severe COVID-19 patients are very breathless (and may be unable to breathe at a comfortable rate on slight moving around or even at rest) and breathe faster than usual, even when sitting still. They cannot finish a sentence without extra breaths. They may even avoid speaking. Their oxygen levels may have fallen so the urge to breathe faster is strong. Physicians usually measure breathing rates when assessing this condition. Normal adults breathe at about 12-18 breaths per minute when they are not thinking about it. In pneumonia the rate rises, sometimes markedly.

The preserved lung compliance in COVID-19 patients suggests that lung epithelial response to SARS-CoV-2 is not the single component of the disease. Moreover, the hyperinflammatory state demonstrated in COVID-19-related ARDS patients, also suggests an endothelial activation consecutive to the inflammatory response as the *primum movens.*

COVID-19 appears to damage the vasculature of the lungs. Particularly, it may induce capillary endothelial cell/microvascular dysfunction which may cause individual cell necrosis. Histopathologic basis of COVID-19 severe disease cases has been analyzed and showed in all cases diffuse alveolar damages involving activation of megakaryocytes, with platelet aggregation and platelet-rich clot formation, in addition to fibrin deposition.

Typically, COVID-19 patients are detected by a consistent clinical history, epidemiological contact, and a positive SARS-CoV-2 test. Specifically, SARS-CoV-2 infection can be confirmed by positive detection of viral RNA in nasopharyngeal secretions using a specific PCR test.

Also, according to the Berlin 2012 ARDS diagnostic criteria, COVID-19 ARDS is diagnosed when a COVID-19 patient presents (1) acute hypoxemic respiratory failure, (2) within 1 week of worsening respiratory symptoms; (3) bilateral airspace disease on chest x-ray, computed tomography, or ultrasound that is not fully explained by effusions, lobar or lung collapse, or nodules; and (4) cardiac failure is not the primary cause of acute hypoxemic respiratory failure. It also found that shortness of breath - also known as dyspnoea - is the only symptom of COVID-19 that is significantly associated with severe cases and with patients requiring admission to ICU. COVID-19 ARDS follows a predictable time course over days, with median time to intubation of 8.5 days after symptom onset. It is therefore important to monitor patients for the development of ARDS as their COVID-19 progresses. The respiratory rate and SpO₂ are two important parameters for judging patients' clinical condition and allowing early recognition of ARDS. A patient who fits any one of the following conditions may have severe disease and requires further evaluation/Respiratory rate ≥ 30 breaths/min; SpO₂ ≤ 92 %; PaO₂/FiO₂ ≤ 300 mmHg.

According to the present invention, methods of treating and compositions for use in a method according, may further comprise administering an anti-IL6 monoclonal antibody or an anti-IL6 receptor monoclonal antibody. By way of anti-IL6 monoclonal antibody or an anti-IL6 receptor monoclonal antibody which can be administered to a COVID-19 patient in combination with at least antagonist or inhibitor of chemokine receptor CXCR4, we may cite siltuximab (an anti-IL6 chimeric monoclonal antibody for treating Castleman's disease, and marketed under the tradename Sylvant®), olokizumab (an anti-IL6 humanized monoclonal antibody under development by the company R-Pharm for treating rheumatoid arthritis and also called CDP6038 or OKZ), sirukumab (an anti-IL6 human monoclonal antibody under development by the company Centocor for treating rheumatoid arthritis and also called CNT0136), tocilizumab (an anti-IL6 human monoclonal antibody marketed by Genentech under the tradename Actemra® for treating rheumatoid arthritis) elsilimomab (an anti-human IL6 therapeutic antibody currently developed by Immunogen), clazakizumab (an anti-IL6 developed by BMS for treating rheumatoid arthritis, previously called BMS-945429 or ALD518), levilimab (BCD-089), CPSI-2364, ALX-0061.

Methods of treating and compositions for use in a method according to the present invention, may further comprise administering a pharmaceutically acceptable vehicle, antiviral drugs, antibacterial drugs, antibiotics, PPI Inhibitors, quinoline compounds, zinc compounds, gamma globulin, hematopoietic cells, mesenchymal cells, anti-inflammatory drugs, vaccines, small interfering RNAs and microRNAs, immunomodulators, or plasma from convalescent patients. Preferably, said methods and compositions for use according to the present invention do not comprise administering any hydroxychloroquine or chloroquine to said COVID-19 patients.

Anti-viral drugs may be selected from the following drugs: nucleotide analogues, nucleoside analogues, nucleoside reverse transcriptase inhibitors (NRTIs), non-nucleoside reverse transcriptase inhibitors (NNRTIs), neuraminidase inhibitors, endonuclease inhibitors, adamantanes, protease inhibitors (PIs), integrase inhibitors (INSTIs), fusion inhibitors (FIs), chemokine receptor antagonists (CCR5 antagonists), or siRNA.

By way of examples of anti-viral drug which may be administered to a COVID-19 patient in combination with the antagonist or inhibitor of chemokine receptor CXCR4 as described above, we may cite abacavir, acyclovir, adefovir, amantadine, ampligen, amprenavir (Agenerase™), arbidol, atazanavir, atripla, balavir, baloxavir marboxil (Xofluza™), biktarvy, boceprevir (Victrelis™), cidofovir, cobicistat (Tybost™), combivir, daclatasvir (Daklinza™), darunavir, delavirdine, descovy, didanosine, docosanol, dolutegravir, doravirine (Pifeltro™), ecoliever, edoxudine, efavirenz, elvitegravir, emtricitabine, enfuvirtide, entecavir, etravirine (Intelence™), famciclovir, fomivirsen, fosamprenavir, foscarnet, fosfonet, favipiravir, triazavirin, ganciclovir (Cytovene™), ibacitabine, ibalizumab (Trogarzo™), idoxuridine, imiquimod, imunovir, indinavir, inosine, interferon type I, interferon type II, interferon type III, lamivudine, letermovir (Prevymis™), lopinavir, loviride, maraviroc, methisazone, moroxydine, nelfinavir, nevirapine, nexavir, nitazoxanide, norvir, oseltamivir (Tamiflu™), PEG interferon α-2a, peginterferon α-2b, penciclovir, peramivir (Rapivab™), pleconaril, podophyllotoxin, pyramidine, raltegravir, remdesivir, ribavirin, rilpivirine (Edurant™), rimantadine, ritonavir, saquinavir, simeprevir (Olysio™), sofosbuvir, stavudine, telaprevir, telbivudine (Tyzeka™), tenofovir alafenamide, tenofovir disoproxil, tenofovir, tipranavir, trifluridine, trizivir, tromantadine, truvada, valaciclovir (Valtrex™), valganciclovir, vicriviroc, vidarabine, viramidine, zalcitabine, zanamivir (Relenza™), zidovudine.

Compositions and methods as described above may be administered via parenteral, oral, nasal, ocular, transmucosal, or transdermal. Most preferred routes of administration are intravenous, intramuscular or subcutaneous routes.

The specific therapeutic dose to be administered to a COVID-19 patient is the dose required to obtain therapeutic and/or prophylactic effects. This dose may be determined by the physician depending on the conditions of the patients, weight, age and sex, compound administered, the route of administration, etc... The dosage may be by a single dose, divided daily dose, or multiple daily doses. Alternatively, continuous dosing, such as for example, via a controlled (*e.g.*, slow) release dosage form can be administered on a daily basis or for more than one day at a time.

According to the preferred embodiment as described above, plerixafor may be selected as antagonist or inhibitor of chemokine receptor CXCR4. It is then administered via the subcutaneous route at a dosage of around 10 to 40 µg/kg bid (20 to 80 µg/kg/day).

According to the present invention, methods and compositions are particularly useful for treating most vulnerable patients that have health conditions or comorbidities, such as hypertension, obesity, diabetes and/or previous myocardial infarction. In addition, compositions and methods as described above are expected to have a protective effect on the heart, since Applicants have showed that administration for example of Plerixafor improves cardiac remodeling quantified by Fulton index.

The COVID-19 patient which may be treated include a patients with previous myocardial infarction, selected from the group consisting of an end stage renal disease (ESRD) patient, a patient having chronic obstructive pulmonary disease (COPD), a cancer patient on immunosuppressive therapy, an AIDS patient, a diabetic patient, a patient subject to obesity, a patient subject to hypertension, a neonate, a transplant patient, a patient on immunosuppression therapy, a patient with malfunctioning immune system, an autoimmune disease patient, an elderly person in an extended care facility, a patient with autoimmune disease on immunosuppressive therapy, a burn patient, and a patient in an acute care setting.

CXCR4 antagonists suitable for use in accordance with the present invention can be administered alone but, in human therapy, will generally be administered in admixture with a suitable pharmaceutically acceptable vehicle, excipient, diluent, or carrier selected with regard to the intended route of administration and standard pharmaceutical practice. Such pharmaceutically acceptable vehicle or excipient may be present in an amount between 0.1% and less than 100% by weight. Optimizing drug-excipient ratios are with the reach of a person with ordinary skill in art for instance the desired weight ratio of drug/excipient in the composition could be less than or equal to the ratio of solubilities of drug/excipient, in a suitable medium.

Pharmaceutical compositions of the present invention may be administered parenterally, for example, intravenously, intraarterially, intraperitoneally, intrathecally, intraventricularly, intrasternally, intracranially, intramuscularly, subcutaneously, or they may be administered by infusion or needle-free techniques.

For such parenteral administration they are best used in the form of a sterile aqueous solution, which may contain other substances, for example, enough salts or glucose to make the solution isotonic with blood. The aqueous solutions should be suitably buffered (preferably, to a pH of from about 3 to 9), if necessary. The preparation of suitable parenteral formulations under sterile conditions is readily accomplished by standard pharmaceutical techniques well known to those skilled in the art.

According to another embodiment of the present invention is directed to a composition for use in a method of treating and a method of treating COVID-19 disease in a subject in need thereof comprising: i) determining a viral load in a sample obtained from the subject by RT-PCR or other equivalent techniques; ii) comparing the viral load determined at step i) with a predetermined reference value; and iii) administering to the subject a therapeutically effective amount of at least one antagonist or inhibitor of chemokine receptor CXCR4 as described above. According to this embodiment, the sample is preferably a blood sample or a mucus sample.

Throughout this application, various references are referred to and disclosures of these publications in their entireties are hereby incorporated by reference into this application to more fully describe the state of the art to which this invention pertains.

### EXAMPLES

### EXAMPLE 1: Effect of CXCR4 antagonist on lung function

Mice which presented lung injury induced by cigarette smoke exposure with poly-IC instillations received daily subcutaneous injections of either PBS or 1 mg/kg plerixafor during the 5 weeks. Lung function was then assessed by measured by the FEV0.05/FVC ratio. Figure 1 showed a statistically significant improvement of the murine lung function after treatment with 1 mg/kg plerixafor injection versus to PBS injection.

### EXAMPLE 2: Effect of CXCR4 antagonist on cardiac function

As in Example 1, mice which presented lung injury induced by cigarette smoke exposure with poly-IC instillations received daily subcutaneous injections of either PBS injection or 1 mg/kg plerixafor during the 5 weeks. Cardiac function and remodeling were then quantified by Fulton index, calculated as RV/(LV+S), with LV: left ventricle, RV: right ventricle, S: septum. Figure 2 showed a statistically significant improvement of the murine cardiac function after treatment with 1 mg/kg plerixafor injection versus to PBS injection.

### EXAMPLE 3: Clinical Trial 1 "LEONARDO"

### pLErixafOr iN Acute Respiratory Distress syndrome related to cOvid-19 (Phase IIb) Example 3.1: Design

Randomized, double blind, placebo-controlled, multicenter trial with parallel groups.

### Example 3.2: Primary objective:

To demonstrate that the CXCR4 antagonist plerixafor on top of the best available treatment is able to increase Ventilator-free days in COVID-19-related ARDS patients.

### Example 3.3: Primary outcome

Ventilator-free days (VFDs) at 28 days are one of several organ failure-free outcome measures to quantify the efficacy of therapies and interventions. VFDs are typically defined as follows:
a. VFDs = 0 if subject dies within 28 days of mechanical ventilation.
b. VFDs = 28 - x if successfully liberated from ventilation x days after initiation.
c. VFDs = 0 if the subject is mechanically ventilated for >28 days.

### Example 3.4: Secondary outcomes:

1. Day-28 mortality [Time Frame: 28 days after randomization] Mortality rate evaluated 28 days after randomization
2. Day-90 mortality [Time Frame: 90 days after randomization], Mortality rate evaluated 90 days after randomization
3. Percentage of patients free of invasive ventilation within 28 days [Time Frame: At Day28]
4. Number of ICU-free days within 28 days [Time Frame: at Day28]
5. CT-scan presence of lung fibrosis at month 6 [Time Frame: at Month 6]
6. Safety at days 1, 7, 14, 28 (pulmonary embolism, myocardial infarction, cardiac arrhythmia, arterial hypotension, anemia, thrombocytopenia, hyperleukocytosis, estimated GFR, proteinuria)
7. Blood levels of CRP, ferritin, D-dimers at Day 1, Day 3 and Day 7 and Day 28

### Example 3.5: Other secondary outcomes in a Sub-study:

1. Blood neutrophils and fibrocytes count at Day 1, Day 3 and Day 7
2. Blood CXCR4-positive neutrophils, CXCR4-positive fibrocytes, CD34⁺ cells count at Day 1, Day 3 and Day 7
3. Blood CXCL12 concentrations at Day 1, Day 3 and Day 7
4. Blood NETosis at Day 1, Day 3 and Day 7
   a. MPO and H3 citrullinated
   b. DNase

### Example 3.6: Inclusion criteria

- Male or female ≥ 18 years of age
- Willing and able to provide written informed consent (or provided by a proxy)
- Currently hospitalized with laboratory confirmed COVID-19 infection
- Requiring invasive mechanical ventilation of less than 48h
- Standard of care recommended by FDA-EMA at the beginning of the trial
- With diagnosis of ARDS according to Berlin criteria

### Example 3.7: Exclusion criteria

- Patient with a do-not-resuscitate order (DNR order)
- Pregnancy or breast feeding
- Known bacterial pneumonia
- Current myocardial infarction
- Known cancer (solid or blood) during the last 5 previous years
- Primitive pulmonary hypertension, lung fibrosis
- Hypersensitivity to Plerixafor
- Neutrophil concentration > 50 000 / mm³ at any time

### Example 3.8: Dosage schedule

Plerixafor subcutaneously 10 to 40 µg/kg bid (20 to 80 µg/kg/day) for 14 days.

In HIV clinical trials, plerixafor (AMD3100) was administered for 10 days by continuous intravenous infusion in an open-label dose escalation study from 2.5 to 160 µg/kg/h.

In WHIM syndrome, plerixafor was administered for 6 months by subcutaneously injection (10 to 20 µg/kg bid for 6 months).

Dose must be adjusted when creatinine clearance < 50 ml/min.

### Example 3.9: Number of patients to be included

Based on recently submitted study, ARDS patients treated by Tocilizumab had: VFDs = 14.86 +/- 5.36 at days 28.

The hypothesis is that plerixafor treatment in combination with best available treatment as described in herein above, will increase VFDs by 2 days. 4 groups of patients (placebo, pleri 10, pleri 20, pleri 40 µg/kg bid) will be planned. To get a power of 90% with an alpha risk of 5%, we have to include 132 patients per arm.

### EXAMPLE 4: Clinical Trial 2 "PLANET"

### PlerixAfor iN modEraTe forms of COVID-19 (Phase IIb)

### Example 4.1: Design

Randomized, double blind, placebo-controlled, multicenter trial with parallel groups.

### Example 4.2: Primary objective:

The choice between choices 1 and 2 will depend on the percentage of patients moving from moderate to severe forms of COVID-19 (data coming soon, DISCOVERY/CORIMMUNO trials)

### Choice 1:

To demonstrate that the CXCR4 antagonist Plerixafor on top of the best available treatment can decrease the time to reach a mild form of COVID-19.

### Choice 2:

To demonstrate that the CXCR4 antagonist Plerixafor on top of the best available treatment is able to decrease the percentage of patients with moderate forms of COVID-19 that are either deceased or need ventilation (mechanical or noninvasive) at day 14.

### Example 4.3: Primary outcome

### Choice 1:

Time to reach mild form of COVID-19, defined by no need of ventilatory support (WHO criteria).

### Choice 2:

Percentage of patients that need ventilation (mechanical or noninvasive) or deceased at day 14

### Example 4.4: Secondary outcomes:

1. Percentage of patients that have a mild form of COVID-19 at day 14
2. Difference between initial CT-scan (diagnosis) and 3-month CT-scan (after randomization) [Time Frame: at Month 3]
3. Difference between initial TDI and final TDI [Time Frame: at Day 14]
4. 6 min walk test distance (m) performed at ambient air at day 14]
5. Percentage of patients with SpO2<90% during 6 min walk test performed at ambient air
6. Day-28 mortality [Time Frame: 28 days after randomization] Mortality rate evaluated 28 days after randomization
7. Number of ICU-free days within 28 days [Time Frame: at Day 28]
8. Maximal oxygen rate within 28 days [Time Frame: at Day 28]
9. Number of days alive outside hospital within 28 days [Time Frame: at Day 28]
10. Number of days alive with oxygen therapy within 28 days [Time Frame: at Day28]
11. Safety at days 1, 7, 14, 28 (pulmonary embolism, myocardial infarction, cardiac arrhythmia, arterial hypotension, anemia, thrombocytopenia, hyperleukocytosis, estimated GFR, proteinuria)
12. Blood levels of CRP, ferritin, D-dimers at Day 1, Day 3 and Day 7 and Day 28

### Example 4.5: Other secondary outcomes in a Sub-study:

13. Blood neutrophils and fibrocytes count at Day 1, Day 3 and Day 7
14. Blood CXCR4-positive neutrophils, CXCR4-positive fibrocytes, CD34⁺ cells count at Day 1, Day 3 and Day 7
15. Blood CXCL12 concentrations at Day 1, Day 3 and Day 7
16. Blood NETosis at Day 1, Day 3 and Day 7
   c. MPO and H3 citrullinated
   d. DNase

### Example 4.6: Inclusion criteria

- Male or female ≥ 18 years of age
- Willing and able to provide written informed consent (or provided by a proxy)
- Currently hospitalized with laboratory confirmed COVID-19 infection
- With moderate form of COVID-19, defined by the need for oxygen therapy between 3 and 5 L/min to obtain a percutaneous oxygen saturation greater than 97% and a respiratory rate <25 breaths/min without the need for invasive ventilation.
- Standard of care recommended by FDA-EMA at the beginning of the trial

### Example 4.7: Exclusion criteria

- Patients hospitalized in ICU
- Patients requiring invasive mechanical ventilation
- Pregnancy or breast feeding
- Known bacterial pneumonia
- Current myocardial infarction
- Current pulmonary embolism
- Known cancer (solid or blood) during the last 5 previous years
- Primitive pulmonary hypertension, lung fibrosis
- Hypersensitivity to Plerixafor
- Neutrophil concentration > 50 000 / mm³ at any time

### Example 4.8: Dosage schedule

Plerixafor subcutaneously 10 to 40 µg/kg bid (20 to 80 µg/kg/day) for 14 days

In HIV clinical trials, plerixafor (AMD3100) was administered for 10 days by continuous intravenous infusion in an open-label dose escalation study from 2.5 to 160 µg/kg/h.

In WHIM syndrome, plerixafor was administered for 6 months by subcutaneously injection (10 to 20 µg/kg bid for 6 months).

Dose must be adjusted when creatinine clearance < 50 ml/min.

## Claims

1. A composition for use in a method of treating COVID-19 comprising administering to a patient a therapeutically effective amount at least one antagonist or inhibitor of chemokine receptor CXCR4.

2. The composition for use in a method according to claim 1, wherein said antagonist or inhibitor of chemokine receptor CXCR4 is an indole-based compound, a N-substituted indole compound, a bicyclam compound, a cyclam mimetic compound, a para-xylyl-enediamine-based compound, a guanidine-based antagonist compound, a tetrahydroquinolines-based compound, or a 1,4-phenylenebis(methylene) compound.

3. The composition for use in a method according to claim 1 or 2, wherein said antagonist or inhibitor of chemokine receptor CXCR4 is chosen among plerixafor (AMD3100), burixafor (TG-0054), JM1657, AMD3329, AMD3465, AMD070, MSX-122, CTCE-9908, WZ811, or BKT-140.

4. The composition for use in a method according to any one of the preceding claims, wherein said antagonist or inhibitor of chemokine receptor CXCR4 is administered via route chosen among parenteral, oral, nasal, ocular, transmucosal, or transdermal.

5. The composition for use in a method according to any one of the preceding claims, wherein said antagonist or inhibitor of chemokine receptor CXCR4 is administered via intravenous, intramuscular or subcutaneous route.

6. The composition for use in a method according to any one of the preceding claims, wherein said antagonist or inhibitor of chemokine receptor CXCR4 is plerixafor and wherein plerixafor is administered via the subcutaneous route at a dosage of around 10 to 40 µg/kg bid (20 to 80 µg/kg/day).

7. The composition for use in a method according to any of the preceding claims, wherein the subject is in moderate to advanced stage of COVID-19 infection.

8. The composition for use in a method according to any of the preceding claims, comprising further treating comorbidity or multiple comorbidity of the patient.

9. The composition for use in a method according to any preceding claims, wherein patients with comorbidity or multiple comorbidity comprises patients with previous myocardial infarction, an obese patient, a patient having hypertension, a patient having chronic obstructive pulmonary disease (COPD), end stage renal disease (ESRD) patient, cancer patient on immunosuppressive therapy, AIDS patient, diabetic patient, neonate, transplant patient, patient on immunosuppression therapy, patient with malfunctioning immune system, autoimmune disease patient, elderly patient in an extended care facility, patient with autoimmune disease on immunosuppressive therapy, burn patient, or patient in an acute care setting.

10. The composition for use in a method according to any one of the preceding claims, further comprising administering a pharmaceutically acceptable vehicle.

11. The composition for use in a method according to any preceding claims, further comprising administering to said COVID-19 patient an anti-IL6 monoclonal antibody or anti-IL6 receptor monoclonal antibody, antiviral drugs, antibacterial drugs, antibiotics, PPI Inhibitors, quinoline compounds, zinc compounds, gamma globulin, hematopoietic cells, mesenchymal cells, anti-inflammatory drugs, vaccines, small interfering RNAs and microRNAs, immunomodulators, or convalescent plasma.

12. The composition for use in a method according to claim 11, wherein the anti-IL6 monoclonal antibody or anti-IL6 receptor monoclonal antibody is chosen among siltuximab, olokizumab, sirukumab, tocilizumab, elsilimomab, clazakizumab, levilimab, CPSI-2364, or ALX-0061.

13. The composition for use in a method according to claim 11, wherein antiviral drugs are chosen among abacavir, acyclovir, adefovir, amantadine, ampligen, amprenavir (Agenerase™), arbidol, atazanavir, atripla, balavir, baloxavir marboxil (Xofluza™), biktarvy, boceprevir (Victrelis™), cidofovir, cobicistat (Tybost™), combivir, daclatasvir (Daklinza™), darunavir, delavirdine, descovy, didanosine, docosanol, dolutegravir, doravirine (Pifeltro™), ecoliever, edoxudine, efavirenz, elvitegravir, emtricitabine, enfuvirtide, entecavir, etravirine (Intelence™), famciclovir, fomivirsen, fosamprenavir, foscarnet, fosfonet, favipiravir, triazavirin, ganciclovir (Cytovene™), ibacitabine, ibalizumab (Trogarzo™), idoxuridine, imiquimod, imunovir, indinavir, inosine, interferon type I, interferon type II, interferon type III, lamivudine, letermovir (Prevymis™), lopinavir, loviride, maraviroc, methisazone, moroxydine, nelfinavir, nevirapine, nexavir, nitazoxanide, norvir, oseltamivir (Tamiflu™), PEG interferon α-2a, peginterferon α-2b, penciclovir, peramivir (Rapivab™), pleconaril, podophyllotoxin, pyramidine, raltegravir, remdesivir, ribavirin, rilpivirine (Edurant™), rimantadine, ritonavir, saquinavir, simeprevir (Olysio™), sofosbuvir, stavudine, telaprevir, telbivudine (Tyzeka™), tenofovir alafenamide, tenofovir disoproxil, tenofovir, tipranavir, trifluridine, trizivir, tromantadine, truvada, valaciclovir (Valtrex™), valganciclovir, vicriviroc, vidarabine, viramidine, zalcitabine, zanamivir (Relenza™), zidovudine.

14. A composition for use in a method of treating COVID-19 in a patient in need thereof comprising:
i) determining a viral load in a sample obtained from the subject by RT-PCR or other equivalent techniques;
ii) comparing the viral load determined at step i) with a predetermined reference value; and
iii) administering to the subject a therapeutically effective amount of at least one antagonist or inhibitor of chemokine receptor CXCR4.

15. Composition for use in a method according to claim 14, wherein the sample is a blood sample or a mucus sample.
